# EUROPEAN PATENT SPECIFICATION

(11) **EP 3 845 629 B1**
(45) Date of publication and mention of the grant of the patent: **25.10.2023**
(21) Application number: 19853919.9
(22) Date of filing: 06.08.2019
(51) Int. Cl.: G01N 27/327, C12Q 1/04, C12Q 1/22

(54) **MEASUREMENT DEVICE AND EVALUATION METHOD**
MESSVORRICHTUNG UND BEURTEILUNGSVERFAHREN
DISPOSITIF DE MESURE ET PROCÉDÉ D'ÉVALUATION

(30) Priority: 29.08.2018 JP 2018160199
(43) Date of publication of application: 07.07.2021
(73) Proprietor: National Institute for Materials Science, Tsukuba-shi, Ibaraki 305-0047 (JP)
(72) Inventor: OKAMOTO Akihiro, Tsukuba-shi, Ibaraki 305-0047 (JP); LI Wei-Peng, Tsukuba-shi, Ibaraki 305-0047 (JP)
(74) Representative: Dehns
(86) International application number: PCT/JP2019/030791
(87) International publication number: WO 2020/044978

(56) References cited:
- WO-A1-2017/006122
- DE-A1-102006 016 326
- US-A1- 2012 244 547
- US-A1- 2014 332 407
- PARK MINJUN ET AL: "Real-time monitoring of microbial activity using hydrogel-hybridized carbon nanotube transistors", SENSORS AND ACTUATORS B: CHEMICAL, vol. 263, 22 February 2018 (2018-02-22), pages 486-492, XP055905608, NL ISSN: 0925-4005, DOI: 10.1016/j.snb.2018.02.137
- WEBSTER T A ET AL: "Monitoring Pseudomonas aeruginosa in culture plates using embedded electrochemical sensors", 2014 40TH ANNUAL NORTHEAST BIOENGINEERING CONFERENCE (NEBEC), IEEE, 25 April 2014 (2014-04-25), pages 1-2, XP032692899, DOI: 10.1109/NEBEC.2014.6972976
- VIGUES, N. et al.: "Electro-addressable conductive alginate hydrogel for bacterial trapping and general toxicity determination", Anal. Chim. Acta., vol. 1036, 26 June 2018 (2018-06-26), pages 115-120, XP055695954,
- MASSAD-IVANIR, NAAMA et al.: "Construction and ] Characterization of Porous Si02/Hydrogel Hybrids as Optical Biosensors for Rapid Detection of Bacteria", Adv. Funct. Mater., vol. 20, 2010, pages 2269-2277, XP001556720, DOI: 10.1002/adfm.201000406

## Description

### Technical Field

The present invention relates to a measurement device and an evaluation method.

### Background Art

In the natural field, microorganisms causing a disease with plants as a host and microorganisms causing an infectious disease with animals including human beings as a host (also referred to as pathogenic microorganisms) have been known.

For example, disease damage on a plant due to microorganisms has been known. In a case where the disease damage occurs, examples of a control method include a method for dispersing a chemical agent or the like and/or for extinguishing plants in a range where disease damage occurs, and in general, it is considered that specifying early the microorganisms that cause the disease damage and specifying early the range in which the disease damage occurs are useful as a method for minimizing the damage of the disease damage.

As such a method, in Patent Literature 1, a "detection method of Sphaerotheca aphanis comprising the following steps: 1) a step of amplifying DNA by using DNA obtained from a strawberry plant body and a primer pair consisting of oligonucleotide represented by SEQ ID NO: 1 and oligonucleotide represented by SEQ ID NO: 2; and 2) a step of detecting a DNA amplification product that is obtained" is described.

In addition, an interest in preventing the infectious disease of human beings due to the microorganisms has increased. In particular, various policies and guidelines with respect to the cleaning and sterilization of the medical instrument after use are provided in order to prevent infection through a medical instrument used in a medical facility. Among them, an endoscope is a medical instrument that is used for an examination or a medical treatment in the body cavity of a biological body, and thus, is required to be thoroughly cleaned and sterilized after use.

For this reason, for example, in Patent Literature 2, an endoscope cleaning management system and an endoscope cleaning management method for reliably ensuring a cleaning level of an endoscope such that a sufficient sterilization effect can be obtained are proposed.

DE 102006016326 relates to a method for determining a microbiological state of a sample by transferring the biological material from the sample to a carrier, positioning the carrier with respect to a sample chamber, which has electrodes, and carrying out an electrochemical measurement.

US 2012/0244547 relates to a biosensor for the detection of airborne biomolecules. When a biomolecule of interest contacts the sensing component a characteristic impedance change is detected which indicates the presence/amount of the biomolecule.

Minjun Park et al (Sensors and Actuators B 263 (2018) 486-492) relates to monitoring microbial activity using hydrogel-hybridized carbon nanotube transistors in which the change in current over time is used to monitor growth activity.

### Citation List

### Patent Literature

Patent Literature 1: JP 2016-220651 A
Patent Literature 2: JP 2012-71028 A

### Summary of Invention

### Technical Problem

According to the method described in Patent Literature 1, powdery mildew occurring in strawberries can be detected, but an operation with specialized knowledge, such as the amplification of the DNA obtained from strawberries as a sample by using a specific primer, is necessary, and it is difficult to detect the disease damage on-site.

In the medical instrument such as an endoscope, in the related art, a cultural method, an adenosine triphosphate (ATP) measurement method, and the like have been known as a method of checking a cleaning effect, but in the case of the cultural method, it takes a lot of time and effort until results are obtained. In addition, in the ATP measurement method that is regarded as preferable in Patent Literature 2, all organic substances (biological substances) are a detection target, and thus, it is difficult to evaluate the presence of pathogenic microorganisms. Further, in order to increase the cleaning effect of the medical instrument such as an endoscope, various attempts for improvement, such as a cleaning device, a cleaning jig, and a cleaning brush, have been still made (for example, refer to JP 2015-150224 A, JP 2015-177914 A, JP 2018-130473 A, JP 2017-202128 A, and the like), but a method capable of evaluating the presence of the pathogenic microorganisms has been required to be developed as a method for checking the effect of cleaning processing to which such a technology is applied.

Therefore, an object of the present invention is to provide a measurement device that is capable of simply providing information for evaluating a risk of developing disease damage on a plant on-site without specialized knowledge of an operator. In addition, another object of the present invention is to provide an evaluation method of a risk of developing disease damage on a plant due to microorganisms.

Furthermore, another object of the present invention is to provide a measurement device that is capable of simply sensing the presence of pathogenic microorganisms in a certain target in which microorganisms causing an infectious disease of animals including human beings may be present, such as a medical instrument. In addition, another object of the present invention is to provide an evaluation method of a cleanliness of a medical instrument.

### Solution to Problem

As result of intensive studies of the present inventors in order to attain the objects described above, it has been found that the objects described above can be attained by the following configurations.

The invention is set out in the appended set of claims. The measurement device according to the present invention comprises: a biosensor; a voltage applying part configured for applying a voltage between electrodes of the biosensor, the biosensor comprising a solid electrolyte containing Riboflavin for trapping at least one type of biological particles having an electron transfer function selected from the group consisting of microorganisms existing on an object to be tested and particles derived from the microorganisms by being brought into contact with the object to be tested, and at least two electrodes, wherein a gap distance between the electrodes is less than or equal to the size of the biological particles and the electrodes are configured such that the solid electrolyte and the electrodes are in contact with each other; a detection part configured for measuring a current value flowing between the electrodes by the electron transfer function of the biological particles bridging the gap between the electrodes when the voltage is applied; and a computing part configured for providing information resulting from the microorganisms by comparing the current value with a reference value stored in advance in which a current value and at least one type of factor selected from the group consisting of the presence or absence of the biological particles, a proliferative state of the microorganisms, and the presence or absence of expression of pathogenicity of the microorganisms are defined.

In the measurement device, the biosensor may be a laminated body in which a substrate with solid electrolyte comprising a first substrate and the solid electrolyte disposed on the first substrate, and a substrate with electrodes comprising a second substrate and at least the two electrodes disposed on the second substrate are laminated such that the solid electrolyte and the electrodes are in contact with each other.

In the measurement device, the solid electrolyte may be hydrogel.

In the measurement device, the shortest distance between the electrodes may be 50 nm to 10 µm.

In the measurement device, at least one of the electrodes may be a comb-shaped electrode having a combteeth shape.

In the measurement device, the information resulting from the microorganisms may be information for evaluating a risk of developing disease damage on a plant due to the microorganisms.

In the measurement device, the biological particles may be derived from at least one type of microorganisms selected from the group consisting of genus Phytophthora, genus Botrytis, genus Plasmopara, genus Sphaerotheca, genus Alternaria, genus Uromyces, genus Ustilago, genus Phakopsora, genus Burkholderia, genus Xanthomonadaceae, genus Xylella, genus Pseudomonas, genus Erwinia, genus Streptomyces, genus Candidatus Liberibacterasiaticus causing a disease damage on Citreae, and genus Candidatus Phlomobacter causing a disease damage on strawberries.

In the measurement device, the disease damage on the plant may be at least one type selected from the group consisting of powdery mildew, a rust disease, a leaf spot, wilt, disease damage on roots and stems, disease damage on ears, rotten fruits, disease damage of seed and soilborne decay, crown wart, witch's broom, and a crinkle leaf.

In the measurement device, the information resulting from the microorganisms may be information for evaluating a cleanliness of a medical instrument.

In the measurement device, the medical instrument may be an endoscope.

The evaluation method according to the present invention comprises: a step of trapping at least one type of biological particles having an electron transfer function on a solid electrolyte containing Riboflavin by bringing the solid electrolyte into contact with an object to be tested, the biological particles being selected from the group consisting of microorganisms existing on the object to be tested and particles derived from the microorganisms; a step of forming a biosensor by preparing a substrate with electrodes comprising a substrate and at least two electrodes disposed on the substrate, a gap distance between the electrodes being less than or equal to the size of the biological particles, and by disposing the solid electrolyte after contact on the substrate with electrodes to be in contact with both of the electrodes; a step of measuring a current value to be obtained by the electron transfer function of the biological particles bridging the gap between the electrodes by applying a voltage between the electrodes; and a step of obtaining information resulting from the microorganisms by comparing the current value with a reference value stored in advance in which a current value and at least one type of factor selected from the group consisting of the presence or absence of the biological particles, a proliferative state of the microorganisms, and the presence or absence of expression of pathogenicity of the microorganisms are defined.

In the evaluation method, the information resulting from the microorganisms may be information for evaluating a risk of developing disease damage on a plant due to the microorganisms.

In the evaluation method, the information resulting from the microorganisms may be information for evaluating a cleanliness of a medical instrument.

In the evaluation method, the medical instrument may be an endoscope.

### Advantageous Effects of Invention

According to one aspect of the present invention, a measurement device that is capable of simply providing information for determining a risk of developing disease damage on a plant on-site without specialized knowledge of an operator can be provided. In addition, an evaluation method of a risk of developing disease damage on a plant due to microorganisms can also be provided.

Furthermore, according to another aspect of the present invention, a measurement device that is capable of simply sensing the presence of pathogenic microorganisms in a certain target in which microorganisms causing an infectious disease of animals including human beings may be present, such as a medical instrument, can be provided. In addition, an evaluation method of a cleanliness of a medical instrument can also be provided.

### Brief Description of Drawings

Fig. 1 is an exploded perspective view of a biosensor that can be used in a measurement device according to an embodiment of the present invention.
Fig. 2 is a front view of a portion of a first electrode and a second electrode in which the first electrode and the second electrode are in contact with a solid electrolyte.
Fig. 3 is a perspective view illustrating a substrate with solid electrolyte comprising a first substrate and the solid electrolyte disposed on the first substrate.
Fig. 4 is a schematic view illustrating a state in which the solid electrolyte is brought into contact with a plant organ.
Fig. 5 is a perspective view of the biosensor that is formed by bonding the substrate with solid electrolyte and a substrate with electrodes together through a spacer.
Fig. 6 is a front view illustrating an example of a body of the measurement device according to the embodiment of the present invention.
Fig. 7 is a partial enlarged view of a periphery of a sensor insertion slot in a state where the biosensor is inserted into the body.
Fig. 8 is a sectional view of A-A' in Fig. 7.
Fig. 9 is a configuration of main electronic components contained in a housing of the measurement device.
Fig. 10 is a schematic graph showing a relationship between a current value obtained from each of a sample D1 and a sample D2 and a voltage applying time (a measurement time) in a case where a predetermined voltage is applied between the electrodes (constant potential measurement).
Fig. 11 is a schematic graph showing a size of a current value with respect to potential differences between the electrodes of a sample D3 and a sample D4 in a case where a sweep voltage is applied between the electrodes.
Fig. 12 is a measurement processing flow of a control computer.
Fig. 13 is a perspective view of a substrate with electrodes of a biosensor that can be used in a measurement device according to a second embodiment of the present invention.
Figs. 15(A) and 15(B) are schematic views illustrating an example of a structure of a tube that is a configuration member of an endoscope.
Fig. 16 is a graph showing a relationship between a concentration (an OD₆₀₀ value) of MR-1 and a slope of a "time-current value" curve when a current value is raised, in a test example.
Fig. 17 is a graph showing measurement results of current values obtained by smearing MR-1 culture solutions having different concentrations on a riboflavin-containing LB agar medium, in the test example.
Fig. 18 is a graph showing a relationship between the concentration (a logarithm of the OD₆₀₀ value) of MR-1 and a time to reach a maximum value of the current value, of the results shown in Fig. 17.
Fig. 19 is a graph showing analysis results of differential pulse voltammetry (DPV), of the results obtained in test example.

### Description of Embodiments

Hereinafter, the present invention will be described in detail.

The following configuration requirement may be described on the basis of a representative embodiment of the present invention, but the present invention is not limited to such an embodiment. In particular, the description of an aspect in which an object to be tested is a plant (more specifically, a plant organ) also applies to an aspect in which the object to be tested is a medical instrument (more specifically, an endoscope), and vice versa.

It is noted that a numerical range represented by using "to" indicates a range including numerical values before and after "to" as a lower limit value and an upper limit value.

### [Measurement Device]

A measurement device according to an embodiment of the present invention is a measurement device, comprising: a biosensor; a voltage applying part configured for applying a voltage between electrodes of the biosensor, the biosensor comprising a solid electrolyte containing Riboflavin for trapping at least one type of biological particles having an electron transfer function selected from the group consisting of microorganisms existing on an object to be tested and particles derived from the microorganisms by being brought into contact with the object to be tested, and at least two electrodes, wherein a gap distance between the electrodes is less than or equal to the size of the biological particles and the electrodes are configured such that the solid electrolyte and the electrodes are in contact with each other; a detection part configured for measuring a current value flowing between the electrodes by the electron transfer function of the biological particles bridging the gap between the electrodes when the voltage is applied; and a computing part configured for providing information resulting from the microorganisms by comparing the current value with a reference value stored in advance in which a current value and at least one type of factor selected from the group consisting of the presence or absence of the biological particles, a proliferative state of the microorganisms, and the presence or absence of expression of pathogenicity of the microorganisms are defined. Hereinafter, a configuration of an example of the measurement device according to the embodiment of the present invention will be described by using the drawings.

### [Biosensor]

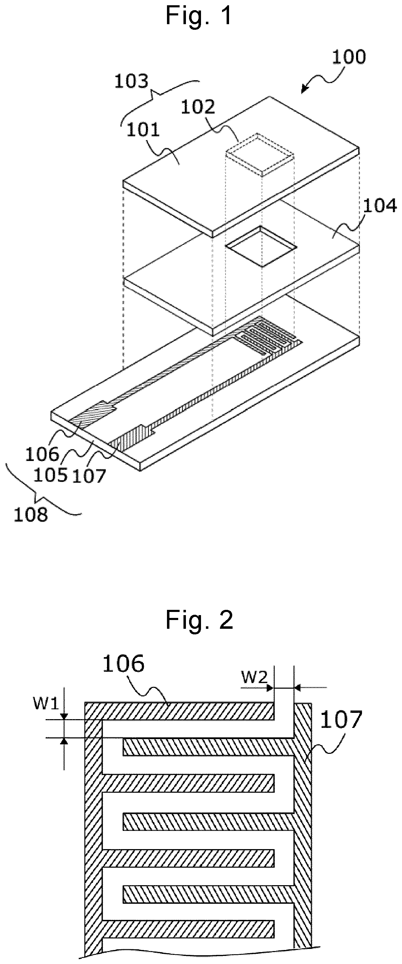

Fig. 1 is an exploded perspective view of a biosensor that can be used in the measurement device according to the embodiment of the present invention. As illustrated in Fig. 1, a biosensor 100 comprises a substrate with solid electrolyte 103 comprising a first substrate 101 and a solid electrolyte 102 disposed on the first substrate (in Fig. 1, disposed on a main surface of the first substrate 101 on the back side) to be in contact with electrodes described below; a spacer 104; and a substrate with electrodes 108 comprising a second substrate 105 and a first electrode 106 and a second electrode 107 disposed on the second substrate 105.

In the biosensor 100, the solid electrolyte 102 is disposed such that the surface is in contact with the first electrode 106 and the second electrode 107 through the spacer 104, but the biosensor that can be applied to the measurement device according to the embodiment of the present invention is not limited to the above-described aspect, and the solid electrolyte may be in contact with the first electrode and the second electrode without the spacer.

Further, as described below, in an aspect in which the solid electrolyte 102 contains a substance that is an electron source and/or an electron transfer substance, the solid electrolyte may be disposed to be in contact with the first electrode and the second electrode disposed on the second substrate of the substrate with electrodes. That is, in such a case, the biosensor does not comprise a spacer and a first substrate, and such a biosensor can also be applied to the measurement device according to the embodiment of the present invention.

In the biosensor 100, the material of the first substrate 101, the spacer 104, and the second substrate 105 is not particularly limited, and in general, a substance having insulating properties (an insulator) is preferable. For example, an organic material, and inorganic material, and the like can be used.

Herein, a substance having electrical resistivity of greater than or equal to 10⁶ (Ω·m) is preferable as the insulator.

The organic material is not particularly limited, and examples thereof include at least any resin selected from the group consisting of a (meth)acrylic resin, a styrene-based resin, a vinylic resin, a polyolefin-based resin, a polyester-based resin, a polyurethane-based resin, a polyamide-based resin, a polycarbonate-based resin, a polydiene-based resin, an epoxy-based resin, a silicone-based resin, a cellulose-based polymer, and chitosan-based polymer; or a copolymer consisting of monomers configuring such resins.

The inorganic material is not particularly limited, and examples thereof include glass.

The biosensor 100 is a two-electrode type sensor. Two electrodes, that is, the first electrode 106 and the second electrode 107 are disposed on the second substrate 105.

The first electrode 106 and the second electrode 107 are an electrode for electrochemically sensing biological particles trapped on the solid electrolyte. A known electrode material can be used, and examples thereof include a metal material such as gold (Au), silver (Ag), platinum (Pt), and palladium (Pd).

Fig. 2 is a front view of a portion of the first electrode 106 and the second electrode 107 in which the first electrode 106 and the second electrode 107 are in contact with the solid electrolyte 102. Each of the first electrode 106 and the second electrode 107 is a comb-shaped electrode having a comb-shaped structure, and has a structure in which the combteeth of the first electrode 106 and the combteeth of the second electrode 107 are alternately disposed.

It is noted that, in Fig. 2, the first electrode 106 has four combteeth, and the second electrode 107 has three combteeth, but the number of combteeth is not limited to the above-described aspect, and can be suitably selected.

In the comb-shaped electrode of Fig. 2, each of the combteeth is disposed at a regular interval of a distance W1, and each of a distance between the tip end of the combteeth of the first electrode 106 and the second electrode 107 and a distance between the tip end of the combteeth of the second electrode 107 and the second electrode 107 (not illustrated) is a distance W2.

In this aspect, the distance W1 and the distance W2 are not particularly limited, and can be suitably selected in accordance with the size of the microorganisms (likely of being) contained in the object to be tested and/or the particles derived from the microorganisms (hereinafter, also referred to as "biological particles to be detected").

According to two electrodes described above, biological particles having a size according to the shortest distance between the first electrode 106 and the second electrode 107 can be detected. Specifically, in the case of Fig. 2, biological particles having a size greater than or equal to the shorter distance of the distances W1 and W2 can be detected.

It is noted that, the shortest distance between the both electrodes is not particularly limited, and in general, 50 nm to 10 µm is preferable.

In other words, it is preferable that the distance between the both electrodes is less than or equal to the size of the biological particles to be detected.

The biological particles to be detected are not particularly limited. In a case where the object to be tested is a plant, at least one type of microorganisms selected from the group consisting of genus Phytophthora, genus Botrytis, genus Plasmopara, genus Sphaerotheca, genus Alternaria, genus Uromyces, genus Ustilago, genus Phakopsora, genus Burkholderia, genus Xanthomonadaceae, genus Xylella, genus Pseudomonas, genus Erwinia, genus Streptomyces, genus Candidatus Liberibacterasiaticus causing a disease damage on Citreae, and genus Candidatus Phlomobacter causing a disease damage on strawberries; and/or particles derived from such microorganisms are preferable.

For example, in a case where a microorganism belonging to the genus Pseudomonas known as causative bacteria of Pith necrosis or the like that occurs in the stem of a tomato are trapped on the solid electrolyte (may be further trapped on the electrode), a current value generated by the microorganism belonging to the genus Pseudomonas is measured, and thus, the presence of the bacteria can be sensed.

As described above, in the measurement device according to the embodiment of the present invention, the biosensor 100 is electrically connected to a body described below, a voltage is applied between the first electrode 106 and the second electrode 107 such that a potential difference occurs between the both electrodes, and the biological particles to be detected trapped on the solid electrolyte are detected.

In this aspect, in a case where the shortest distance between the first electrode 106 and the second electrode 107 is in the range described above, the biological particles having an electron transfer function bridge a gap between the both electrodes in which a potential difference occurs, and thus, a current flows between the both electrodes. That is, the biological particles can be instantly detected by measuring the current value.

Specifically, it is assumed that one of the biological particles described above bridges the gap between the electrodes in which a potential difference of 50 mV occurs, and thus, a current value of approximately 100 pA can be measured.

For example, in a case where the biological particles to be detected are planospores of a fungus belonging to the genus Phytophthora, it is known that the size is approximately 5 to 10 µm. In this aspect, in a case where the shortest distance between the electrodes is in the range described above, for example, is 8 µm, it is assumed that the planospores present on the solid electrolyte bridge the gap between the electrodes such that the current value can be measured, and thus, the planospores may be trapped on the surface of the solid electrolyte, that is, the planospores may be present on the surface of the plant organ, and the surface of the plant organ may be in a state suitable for fungal proliferation.

On the other hand, in this aspect, even in a case where the general bacteria are present on the solid electrolyte, the size of the bacteria is generally sufficiently smaller than the distance between the electrodes described above, and thus, the bacteria are not capable of bridging the gap between the electrodes, and therefore, are not detected as a signal. The shortest distance between the electrodes is adjusted in accordance with the size of the biological particles to be detected, and thus, the biological particles to be detected can be differentially detected.

It is noted that, in the biosensor 100, both of the first electrode 106 and the second electrode 107 are a comb-shaped electrode, but a two-electrode type biosensor used in the measurement device of the present invention is not limited to the above-described aspect, and the shape of the electrode is not particularly limited insofar as the solid electrolyte is disposed such that the solid electrolyte and the both electrodes are in contact with each other. In such an aspect, the shortest distance between the two electrodes is the same as that described in the case of the comb-shaped electrode.

In addition, it is preferable that at least one selected from the group consisting of the first electrode and the second electrode is a comb-shaped electrode, as the two-electrode type biosensor used in the measurement device according to the embodiment of the present invention. In a case where at least one of two electrodes is a comb-shaped electrode, a larger current value is easily obtained, and thus, the measurement can be more rapidly and/or more accurately performed.

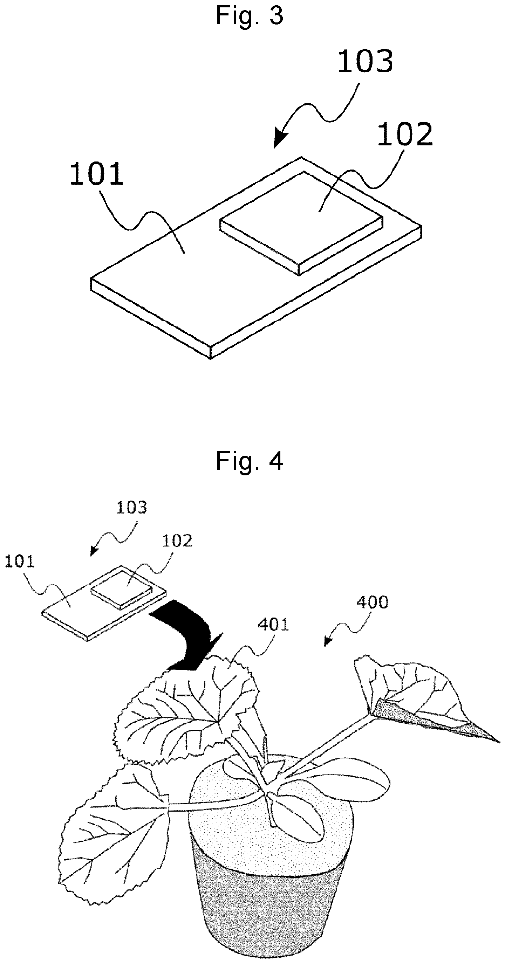

Fig. 3 is a perspective view illustrating the substrate with solid electrolyte 103 comprising the first substrate 101 and the solid electrolyte 102 disposed on the first substrate 101.

The first substrate 101 described above is a member having a function of supporting the solid electrolyte 102, and is in the shape of a sheet in Fig. 3, but the shape is not particularly limited insofar as the first substrate 101 is capable of supporting the solid electrolyte 102.

The material of the first substrate 101 is not particularly limited, an insulator is preferable, and examples of the material of the first substrate include an organic material and an inorganic material. Examples of the organic material include paper and high molecules. Examples of the inorganic material include glass.

Furthermore, the thickness of the first substrate is not particularly limited, and can be suitably selected, and typically, 0.1 µm to 10 mm is preferable from the viewpoint of easy handling.

The solid electrolyte 102 is a member for trapping at least one type of biological particles selected from the group consisting of the microorganisms existing on an object to be tested and the particles derived from the microorganisms (for example, spores) by being brought into contact with the object to be tested.

The object to be tested is not particularly limited, and for example, a medium necessary for controlling disease damage on a plant, a medium necessary for preventing an infectious disease due to the microorganisms in animals including human beings, and the like can be the object to be tested.

Examples of the object to be tested include a plant organ (for example, a leaf, a stem, a petal, a root, and the like), the soil and the air in a growth environment of a plant (for example, the atmospheric air of an open-agricultural field and/or a greenhouse-agricultural field), and water (water for watering, aerosol, and the like).

In addition, examples of the object to be tested include a medical instrument, and more specifically, a configuration member of the medical instrument that is in contact with a biological mucous membrane or the like of a subject during use. For example, in the case of an endoscope, an insertion portion that is inserted into the subject (a tip end portion, a bending portion, a passive bending portion, a flexible tube portion, and the like), an operation portion connected to the insertion portion (an angle knob, an air/water supply cylinder, an air/water supply button, and the like), an endoscope connector connected to the tip end of a universal cord extending from the operation portion (a water line, an auxiliary water ferrule, and the like), and a cleaning brush, a cleaning /sterilization waste liquid, and the like for such members. The configuration member, the structure, and the like of the endoscope are known, and JP 2018-130473 A or the like is referred to, as necessary.

A method for bringing the solid electrolyte 102 into contact with the object to be tested is not particularly limited, and may be suitably selected in accordance with the type of object to be tested.

The solid electrolyte 102 is a member to be a medium for electrochemical measurement described below, and a known solid electrolyte can be used as the material thereof. Among them, hydrogel is preferable as the solid electrolyte, from the viewpoint of excellent handleability. Examples of the hydrogel include agar gel and gelatin gel.

It is noted that a solid electrolyte having high ions conductivity is preferable as the solid electrolyte, and more specifically, a solid electrolyte is preferable in which ions (for example, hydrogen ions, sulfate ions, and the like) can be moved.

The hydrogel described above may contain a sulfate salt or the like, from the viewpoint of having more excellent ions conductivity.

In addition, the solid electrolyte contains Riboflavin which is an electron transfer substance, in order to aid or accelerate the electron transfer function of the biological particles to be detected. An organic compound is preferable as the substance that is an electron source, and examples thereof include glucose, an acetic acid, and a lactic acid..

Furthermore, a protective sheet (not illustrated) may be further disposed on the solid electrolyte 102. In a case where the protective sheet is disposed on the solid electrolyte, the solid electrolyte is used by peeling the protective sheet at the time of use, and thus, a clean state of the solid electrolyte can be maintained until use, and a more accurate measurement result can be easily obtained.

### (Specific Embodiment 1 of Object to be tested)

Hereinafter, a contact method with respect to the solid electrolyte will be described by using a case where the object to be tested is a plant organ, as an example.

Fig. 4 is a schematic view illustrating a state in which the solid electrolyte is brought into contact with the plant organ. The solid electrolyte 102 is brought into contact with a leaf 401 of a plant 400. A method for bringing the solid electrolyte 102 into contact with the leaf 401 is not particularly limited, and the solid electrolyte 102 may be brought into contact with at least one spot of the surface of the leaf 401. As described above, at least a part of the biological particles present on the leaf 401 is trapped on the solid electrolyte 102.

It is noted that, in Fig. 4, the solid electrolyte 102 is brought into contact with the leaf 401 of the plant 400, and the solid electrolyte described above may be brought into contact with a desired organ, for example, may be brought into contact with an arbitrary organ such as a stem, a leaf, a root, and a petal.

The plant that is the object to be tested in the measurement device according to the embodiment of the present invention is not particularly limited, and examples thereof include vegetables, cereal crops, edible fruits, and algae.

Examples of the vegetables include Japanese basil, strawberries, watermelons, melons, daikon radishes, carrots, burdock roots, Irish potatoes, sweet potatoes, onions, asparagus, cabbages, lettuces, spinach, napa cabbages, tomatoes, eggplants, pumpkins, bell peppers, cucumbers, Japanese ginger, cauliflowers, broccoli, edible chrysanthemum, ginger, and turmeric.

Examples of the cereal crops include corns, rice, wheat, barley, oat, rye, millet, Italian millet, Japanese millet, sorghum (Sorghum bocolor, Sorghum nervosum, and Sorghum bicolor), soybean, azuki bean, mug bean, marrow bean, peanut, pea, horse bean, lentil, chickpea, buckwheat, and Bitter buckwheat.

Examples of the edible fruits include oranges, grapefruits, Yuzu, Japanese bitter oranges, Kabosu oranges, Sudachi oranges, hirami lemons, lemons, limes, citrons, Japanese summer oranges, Hassaku oranges, new summer oranges, Jabara oranges, Oroblanco oranges, Iyokan oranges, Mandarin oranges, Unshiu oranges, Ponkan oranges, Kishu oranges, Chinese oranges, Chinese quinces, Chinese white pears, nashi pears, medlars, apples, American cherries, apricots, Japanese apricots, cherries, prunes, peaches, almonds, ginkgoes, Japanese chestnuts, walnuts, pecans, chocolate vines, figs, giant pacific oysters, blackcurrants, red raspberries, kiwi fruits, silverberries, mulberries, cranberries, cowberries, pomegranate fruits, currants, jujubes, honeyberries, bilberries, grapes, blackberries, blueberries, raspberries, olives, loquats, and bayberries.

Examples of the algae include seaweeds such as green algae, red algae, and ulvophytes; and blue-green algae (cyanobacteria) and euglena.

As illustrated in Fig. 1, the substrate with solid electrolyte is bonded to the substrate with electrodes through the spacer to face each other, after being brought into contact with the plant organ. At this time, the position of each of the substrates is adjusted such that the solid electrolyte and two electrodes are in contact with each other.

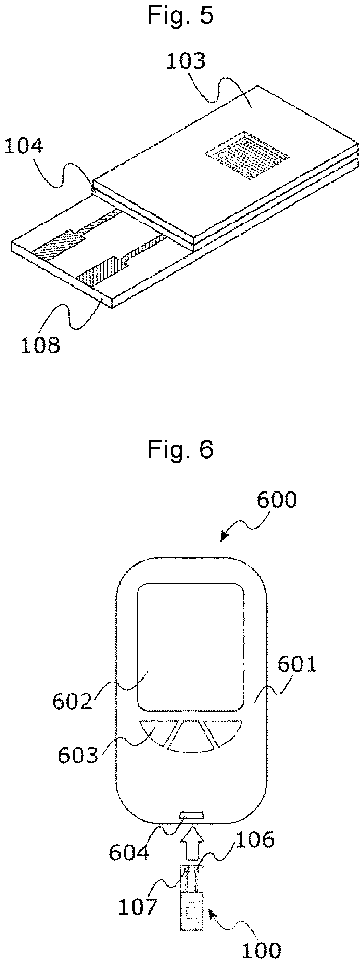

It is noted that Fig. 5 is a perspective view of the biosensor 100 that is formed by bonding the substrate with solid electrolyte 103 and the substrate with electrodes 108 together through the spacer 104. In the biosensor 100, two electrodes of the substrate with electrodes 108 and the solid electrolyte of the substrate with solid electrolyte 103 are in contact with each other.

In other words, the biosensor is a laminated body in which the substrate with solid electrolyte comprising the first substrate and the solid electrolyte disposed on the first substrate, and the substrate with electrodes comprising the second substrate and two electrodes disposed on the second substrate are laminated such that the solid electrolyte two or more electrodes are in contact with each other.

An approach to obtain information for evaluating a risk of developing disease damage on a plant or information for evaluating a cleanliness of a medical instrument as described below, by using the biosensor having the configuration as described above, is completely different from a biochemical approach with respect to the similar object to be tested in the related art, and takes an advantage of a physicochemical method based on electrochemical measurement, and thus, it can be said that the approach is a completely new approach using the electron transfer function of the microorganisms, even from the viewpoint of the application of the same type of biosensor.

Fig. 6 is a plan view illustrating an example of a body 600 of the measurement device according to the embodiment of the present invention. The body 600 includes a housing 601, a display 602 arranged on the housing 601, an operation button 603, and a sensor insertion slot 604, and is used by inserting the biosensor 100 into the sensor insertion slot 604.

The body 600 includes a circuit substrate on which an electronic component necessary for a predetermined operation of the measurement device according to the embodiment of the present invention (for example, the applying of a voltage, the communication with respect to the outside, and the like), such as a central processing unit (CPU), a random access memory (RAM), and a read only memory (ROM), is mounted.

It is noted that, in Fig. 6, the body 600 is portable, but the body according to the embodiment of the present invention is not limited to the above-described aspect, and may be stationary.

The display 602 displays a measurement result, and also displays a setting procedure, an operation procedure, an operation situation, and the like. The display 602, for example, is a display device such as a liquid crystal panel, a plasma display panel, and an electroluminescence panel.

A plurality of operation buttons 603 are used for performing an operation such as various settings (the setting of a measurement condition, the input of an ID of a sample, and the like), the start of the measurement, and the end of the measurement. The plurality of operation buttons 603 may be a contact type touch panel. In such a case, the display 602 and the operation button 603 may be integrated.

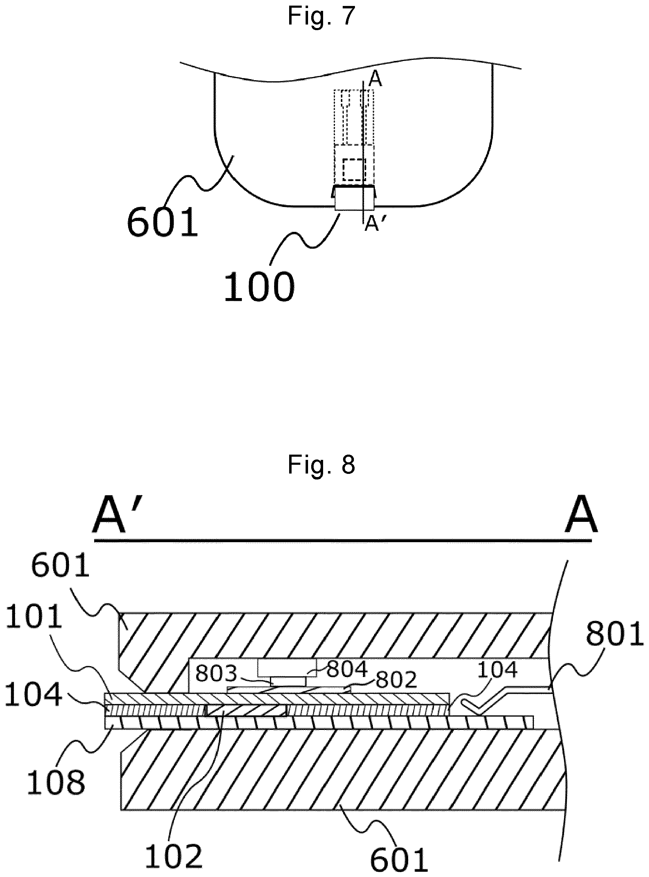

Fig. 7 is a partial enlarged view of the periphery of the sensor insertion slot in a state where the biosensor 100 is inserted into the body 600. In addition, Fig. 8 is a sectional view of A-A' in Fig. 7.

As illustrated in Fig. 7 and Fig. 8, in a case where the biosensor 100 is inserted into the sensor insertion slot 604, the electrode of the biosensor and a connector 801 are electrically connected to each other.

In addition, the inserted biosensor 100 is pressed by an electrical cylinder (an actuator) comprising a plate 802, an extendable rod 803 connected to the plate 802, and a cylinder 804, and thus, the solid electrolyte 102 and the electrodes are in close contact with each other. The measurement device according to the embodiment of the present invention comprises the electrical cylinder therein, the solid electrolyte and the electrodes of the biosensor are pressed with a predetermined pressure to be in close contact with each other, and thus, the measurement condition is easily uniformized for each biosensor, and thus, a variation in the measurement results easily decreases.

It is noted that the measurement device according to the embodiment of the present invention may not comprise the electrical cylinder. In such a case, the biosensor may bond two substrates together to be in close contact with other by using an actuator outside the device, or may bond two substrates together to be in close contact with each other, for example, by the hand working of an operator, without using the actuator.

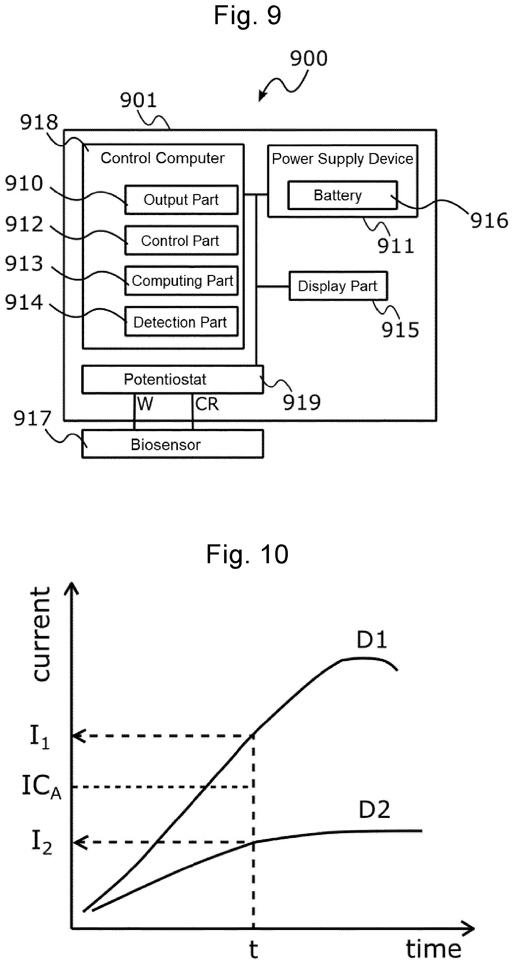

Next, the function of the measurement device according to the embodiment of the present invention will be described. Fig. 9 illustrates the configuration of main electronic components contained in a housing of a measurement device 900. A control computer 918, a potentiostat 919 (corresponding to the voltage applying part), and a power supply device 911 are arranged on a substrate 901 contained in the body.

The control computer 918 comprises a processor such as a central processing unit (CPU), a recording medium such as a memory (a random access memory (RAM) and a read only memory (ROM)), and a communication unit, in a hardware manner, and functions as a device comprising an output part 910, a control part 912, a computing part 913, and a detection part 914 by allowing the processor to load a program stored in the recording medium on the RAM and to execute the program. Furthermore, the control computer 918 may further comprise an auxiliary storage device such as a semiconductor memory (for example, a flash memory) and a hard disk.

The control part 912 controls a timing for applying a voltage to a biosensor 917, an applied voltage value, the driving of the electrical cylinder, and the like. The power supply device 911 comprises a battery 916, and supplies power for operation to the control computer 918 and the potentiostat 919. It is noted that the power supply device 911 can be placed outside the housing.

The potentiostat 919 is capable of setting a potential between two electrodes to be constant or of sweeping a voltage to be applied, is controlled by the control part 912, applies a predetermined voltage between typically two electrodes of the biosensor 917 by using terminals CR and W, measures a response current, and sends a measurement result of the response current to the detection part 914.

The computing part 913 calculates and provides information for evaluating a risk of developing disease damage on a plant by comparing a reference value stored in advance in the storage device arranged on the substrate, with the obtained current value.

In the reference value, at least one type of factor selected from the group consisting of the presence or absence of the biological particles, a proliferative state of the microorganisms, and the presence or absence of the expression of pathogenicity of the microorganisms, and a current value are defined.

The comparison method of the computing part will be described by using Fig. 10. Fig. 10 is a schematic graph showing a relationship between a current value obtained from each of a sample D1 and a sample D2 and a voltage applying time (a measurement time) in a case where a predetermined voltage is applied between the electrodes (constant potential measurement).

The reference value in Fig. 10 is the size of a current value at a predetermined measurement time, and is stored as a reference value IC_{A} that is set in advance by machine learning or the like, in relation to the presence or absence of the biological particles to be detected on the solid electrolyte (the detection target is selected by the distance between two electrodes) and an expression state of pathogenicity.

Here, the sample D1 is measured, and in a case where the detected current value is I₁ at a time t, the computing part compares the value with the reference value IC_{A}, and outputs the information that predetermined disease damage may have occurred on the plant that is the object to be tested.

It is noted that the output contents, for example, may be a difference value between I₁ and IC_{A}, or may be both of the difference value and information relevant to a possibility that the disease damage occurs on the plant. In addition, a difference value between I₁ and/or I₁ and IC_{A} can be used as an evaluation marker for a risk of developing the disease damage on the plant.

On the other hand, the sample D2 is measured, and in a case where the detected current value is I₂ at the time t, the computing part compares the value with the reference value IC_{A}, and outputs the information that the predetermined disease damage may not have occurred on the plant that is the object to be tested. In this case, the output contents are as described above.

The reference value in Fig. 10 is the size of the current value at the predetermined measurement time, but the reference value in the measurement device according to the embodiment of the present invention is not limited to the above-described aspect, and for example, may be the absolute value of the current value, a change rate of the current value (dl/dt, a first order differential value), a second order differential value (d²l/dt²), an inflection point of dl/dt (a time until the current value is raised), the maximum value of the current value, a time to reach the maximum value of the current value, and the like.

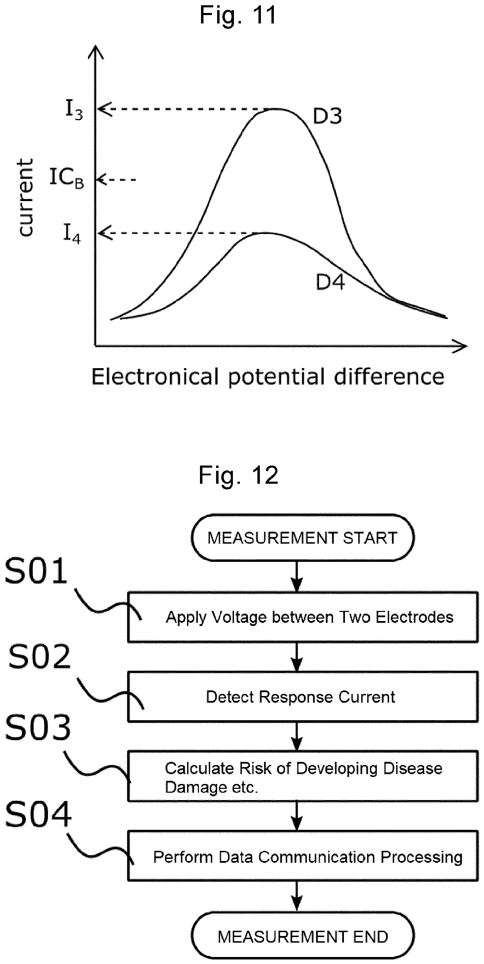

In addition, as another embodiment, Fig. 11 is a schematic graph showing the size of a current value with respect to potential differences between the electrodes of a sample D3 and a sample D4 in a case where a sweep voltage is applied between the electrodes.

Here, the sample D3 is measured, and in a case where the maximum value of the current value detected with respect to the sweep voltage is I₃, the computing part compares the value with a reference value ICs, and outputs the information that the predetermined disease damage may have occurred on the plant that is the object to be tested.

It is noted that the output contents, for example, may be a difference value between I₃ and IC_{B}, or may be both of the difference value and the information relevant to the possibility that the disease damage occurs on the plant. A difference value between I₃ and/or I₃ and ICs can be used as an evaluation marker for a risk of developing the disease damage on the plant.

On the other hand, the sample D4 is measured, and in a case where the detection the maximum value of the current value detected with respect to the sweep voltage is I₄, the computing part compares the value with the reference value ICs, and outputs the information that the predetermined disease damage may not have occurred on the plant that is the object to be tested. In this case, the output contents are as described above.

The reference value in Fig. 11 is the maximum value of the currents to be obtained with respect to the sweep voltage, but the reference value in the measurement device according to the embodiment of the present invention is not limited to the above description, and for example, may be the number of peaks and/or the width of the peak.

The computing part 913 stores the obtained computation result. The output part 910 performs data communication with respect to a display part 915, and transmits a computation result of the computing part 913 for a risk of developing the disease damage on the object to be tested to the display part 915. The display part 915, for example, is capable of displaying the type of disease damage that may have occurred on the plant that is the object to be tested and the computation result such as the progress situation thereof, which are received from the output part 910, on a display screen in a predetermined format.

Fig. 12 illustrates a measurement processing flow of the control computer 918.

In a case where a measurement start instruction is received, the CPU (the control part 912) of the control computer 918 applies a predetermined voltage or a sweep voltage between two electrodes by controlling the potentiostat 919, and starts the measurement of the response current (step S01). It is noted that the sensing of the attachment of the biosensor 917 with respect to the measurement device may be the measurement start instruction. In addition, the control part may bring the solid electrolyte into contact with the electrodes by controlling the electrical cylinder before the start of the measurement.

Next, the potentiostat 919 measures the response current to be obtained by applying the voltage, and sends the measured current to the detection part 914 (step S02).

The computing part 913 performs computation processing, on the basis of the current value, and calculates the content of the biological particles to be detected on the solid electrolyte (not illustrated) of the biosensor 917 (step S03). For example, the computing part 913 of the control computer 918 compares the current value with the reference value by the method described above, and calculates a risk of developing the disease damage on the plant.

The output part 910 transmits a calculation result of the content of the biological particles to be detected to the display unit 915 through a communication link formed between the output unit 910 and the display part 915 (step S04). After that, the control part 912 displays the content of the biological particles to be detected on the display part, and ends the measurement.

Examples of a method for evaluating a risk of developing disease damage typically on a plant due to microorganisms by using the measurement device described above include the following method.

The method is an evaluation method, comprising: a step of trapping at least one type of biological particles having an electron transfer function on a solid electrolyte containing Riboflavin by bringing the solid electrolyte into contact with an object to be tested (typically, a plant organ), the biological particles being selected from the group consisting of microorganisms existing on the object to be tested and particles derived from the microorganisms (a step A); a step of forming a biosensor by preparing a substrate with electrodes comprising a substrate and at least two electrodes disposed on the substrate, a gap distance between the electrodes being less than or equal to the size of the biological particles, and by disposing the solid electrolyte after contact on the substrate with electrodes to be in contact with both of the electrodes (a step B); a step of measuring a current value to be obtained by the electron transfer function of the biological particles bridging the gap between the electrodes by applying a voltage between the electrodes (a step C); and a step of obtaining information resulting from the microorganisms (e.g. for evaluating a risk of developing disease damage due to the microorganisms) by comparing the current value with a reference value stored in advance in which a current value and at least one type of factor selected from the group consisting of the presence or absence of the biological particles, a proliferative state of the microorganisms, and the presence or absence of expression of pathogenicity of the microorganisms are defined (a step D).

According to the method described above, information for evaluating a risk of developing disease damage on a plant can be simply obtained on-site without specialized knowledge of the operator.

The step A, for example, can be implemented by taking the substrate with solid electrolyte described above in an environment where plants are growing.

Further, objective biological particles can be detected by adjusting the shortest distance between the electrodes of the substrate with electrodes used in the step B. The substrate with electrodes can be disposable, and in a case where the measurement is performed by changing the shortest distance between the electrodes, a plurality of types of biological particles (derived from a plurality of microorganisms) can be detected.

In addition, the step C and the step D are implemented by inserting the formed biosensor into the body described above, and by operating the operation button, in accordance with an instruction to be displayed on the display, and thus, can be directly implemented in a place where the step A is implemented insofar as the body is taken in the place, and special preprocessing or a device operation is not necessary, and thus, an operator without specialized knowledge can also simply obtain the information for evaluating a risk of developing the disease damage.

### (Second Embodiment of Measurement Device)

In a case where the object to be tested is a plant organ, a measurement device according to a second embodiment of the present invention is a measurement device, comprising: a biosensor; a voltage applying part configured for applying a voltage between two electrodes of the biosensor, the biosensor comprising a solid electrolyte containing Riboflavin for trapping biological particles on at least a surface of a plant organ by being brought into contact with the organ, two electrodes, and third electrodes different from the two electrodes, and being configured such that the solid electrolyte, the two electrodes, and the third electrodes are in contact with each other; a detection part configured for measuring a current value flowing between the two electrodes when the voltage is applied; and a computing part configured for providing information for determining risk of developing disease damage on the plant by comparing the current value with a reference value stored in advance in which a current value and a risk of developing the disease damage are defined.

Hereinafter, the measurement device according to the second embodiment of the present invention will be described, and the matters not described below are the same as those of the measurement device according to the embodiment illustrated in Fig. 1.

In the measurement device according to this embodiment, the biosensor to be used comprises a substrate with electrodes comprising two electrodes and the third electrodes different from the electrodes described above. That is, the biosensor is a three-electrode type sensor.

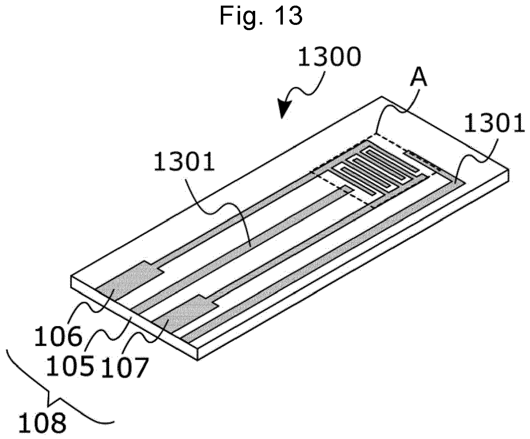

Fig. 13 is a perspective view of the substrate with electrodes of the biosensor. A substrate with electrodes 1300 comprises the first electrode 106 and the second electrode 107 formed on the second substrate 105. Such a configuration is the same as that of the biosensor described above.

The substrate with electrodes 1300 further comprises two third electrodes 1301 on the second substrate 105. It is noted that the substrate with electrodes 1300 comprises two third electrodes 1301, but the biosensor of the measurement device according to this embodiment is not limited to the above-described aspect, and may comprise one third electrode, or may comprise three or more third electrodes.

The third electrode described above functions as a reference electrode in a case where the biosensor is electrically connected to the measurement device. The measurement device comprising the biosensor comprising the reference electrode is capable of measuring an electrode potential, and thus, obtains a more excellent effect of the present invention. It is noted that a known reference electrode for an electrochemical measurement cell can be used as the third electrode, and for example, a silver/silver chloride electrode or the like can be used.

The biosensor is formed by bringing the substrate with electrodes 1300 into contact with the solid electrolyte, and at this time, the solid electrolyte is disposed as illustrated in a region indicated by A in Fig. 13, that is, such that the solid electrolyte is in contact with all of the first electrode, the second electrode, and the third electrode. By disposing the solid electrolyte as described above, more accurate measurement can be performed.

In addition, according to the configuration as described above, impedance measurement can also be performed, and information relevant to the total number of microorganisms on the solid electrolyte can also be obtained.

### (Specific Embodiment 2 of Object to be tested)

Hereinafter, a case where the object to be tested is a medical instrument will be described. It is noted that the matters not described below are the same as those of the embodiments described above and the case where the object to be tested is a plant organ.

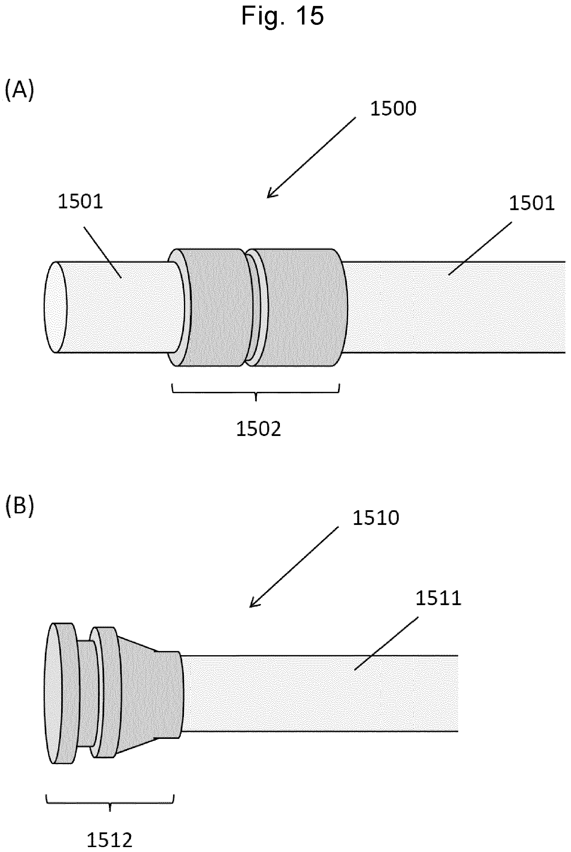

Fig. 15(A) is a schematic view illustrating an example of the structure of a tube configuring a flexible tube portion of an insertion portion to be inserted into a subject, in an endoscope, as a configuration member of a medical instrument. A tube 1500 illustrated in Fig. 15(A) comprises a tube body 1501 and a joint portion 1502. The material of the tube body 1501, for example, is a fluorine resin, silicon rubber, and the like. The material of the joint portion 1502, for example, is a metal such as stainless steel, a resin, and the like.

The tube 1500 is cleaned by a cleaning liquid after the endoscope is used. The solid electrolyte is brought into contact with the outer surface of the tube body 1501 and/or and the joint portion 1502 after cleaning. In addition, in a case where the inner surface of the tube body 1501 and/or the joint portion 1502 is cleaned by using a brush or the like, the solid electrolyte may be brought into contact with the brush or the like. Alternatively, the solid electrolyte may be brought into contact with a waste liquid after cleaning, and in such a case, as necessary, the waste liquid may be subjected to processing such as concentrating in advance.

A method for bringing the solid electrolyte into contact with the medium described above is not particularly limited, and the solid electrolyte may be brought into contact with at least one spot of the surface of the medium (in the case of a liquid, a liquid surface). As described above, at least a part of the biological particles present on the medium is trapped on the solid electrolyte.

Fig. 15(B) is a schematic view illustrating another example of the structure of the tube that is the configuration member of the endoscope. A tube 1510 illustrated in Fig. 15(B) comprises a tube body 1511 and a ferrule portion 1512. The material of the tube body 1511, for example, is a fluorine resin, a silicon rubber, and the like. The material of the ferrule portion 1512, for example, is a metal such as stainless steel, a resin, and the like.

As with the tube 1500 illustrated in Fig. 15(A), in such a tube 1510, the solid electrolyte is brought into contact with an arbitrary medium, and thus, at least a part of the biological particles present on the medium can be trapped on the solid electrolyte.

### [Test Example]

Electrochemical measurement was performed by using the biosensor used in the measurement device according to the embodiment of the present invention, in accordance with the following procedure.

Shewanella oneidensis (S. oneidensis MR-1) (hereinafter, also simply referred to as "MR-1") was used as the biological particles to be detected.

Iron-reducing bacteria represented by a bacterium belonging to the genus Shewanella such as Shewanella oneidensis (S. oneidensis) are bacteria present in the natural field (environmental bacteria), and is known as to regenerate NAD+ by extracellular electron transport (EET). Such bacteria are also referred to as "current generating bacteria" since the bacteria have a function of transferring electrons generated by the decomposition (metabolism) of an electron donor (an organic substance) in the fungus body to an electron acceptor (a metal or the like) outside the fungus body through electron transfer enzyme (cytochrome c) expressed in the extracellular membrane.

In addition, recently, it has been clear that not only bacteria known as current generating bacteria such as bacteria belonging to the genus Shewanella but also pathogenic microorganisms have an electron transfer function.

Accordingly, verified data relevant to the effectivity of the electrochemical measurement using the solid electrolyte on which the biological particles to be detected are trapped can be acquired and considered by a test using MR-1 that is current generating bacteria.

An LB agar medium of 1.5% (w/v) was used as the solid electrolyte, and 30 µL of a culture solution containing MR-1 was smeared on one surface.

It is noted that the culture solution contains a synthetic culture medium containing a lactic acid of 10 mM, and MR-1 exhibits an electron transfer function by the metabolism of a lactic acid or an organic substance contained in the LB agar medium, in an anaerobic condition, and thus, a current is generated.

A three-electrode type electrode configuration (Working Electrode: Gold (a diameter of 4 mm), Counter Electrode: Gold, Reference Electrode: Silver) was adopted as the biosensor, and in this test example, a commercially available screen-printed gold electrode (Model Number: 220AT, manufactured by DropSens S.L.) was used.

Here, it should be noted that the electrode of the three-electrode type biosensor used in this test example is not a comb-shaped electrode having a combteeth shape.

The LB agar medium in which the MR-1 culture solution was smeared on the surface was disposed such that the LB agar medium was in contact with the working electrode and the counter electrode of the biosensor, and a voltage of 0.2 V was applied in an anaerobic condition, and thus, electrochemical measurement was performed.

### (Measurement Using MR-1 Culture Solutions Having Different Concentrations)

A current value (µA) for 24 hours from the start of the measurement (a time point when a voltage was applied to the electrode of the biosensor) was measured by using a culture solution having a light absorbance at a wavelength of 600 nm (OD₆₀₀) of 0 (a control not containing MR-1), 0.01 (7.5 × 10⁵ cells), 0.05 (3.75 × 10⁶ cells), 0.1 (7.5 × 10⁶ cells), 0.4 (3 × 10⁷ cells), and 0.8 (6 × 10⁷ cells), as the culture solution to be smeared on the LB agar medium (results are not included in the drawings).

It is noted that, in the previously reported literature (PLoS ONE 2015, 10(6): e0131249), an MR-1 culture solution is described as approximately 5 × 10⁸ cells per 1 mL of the culture solution when the OD₆₀₀ value was 0.1, and thus, it was approximately the same as the MR-1 culture solution in this test example.

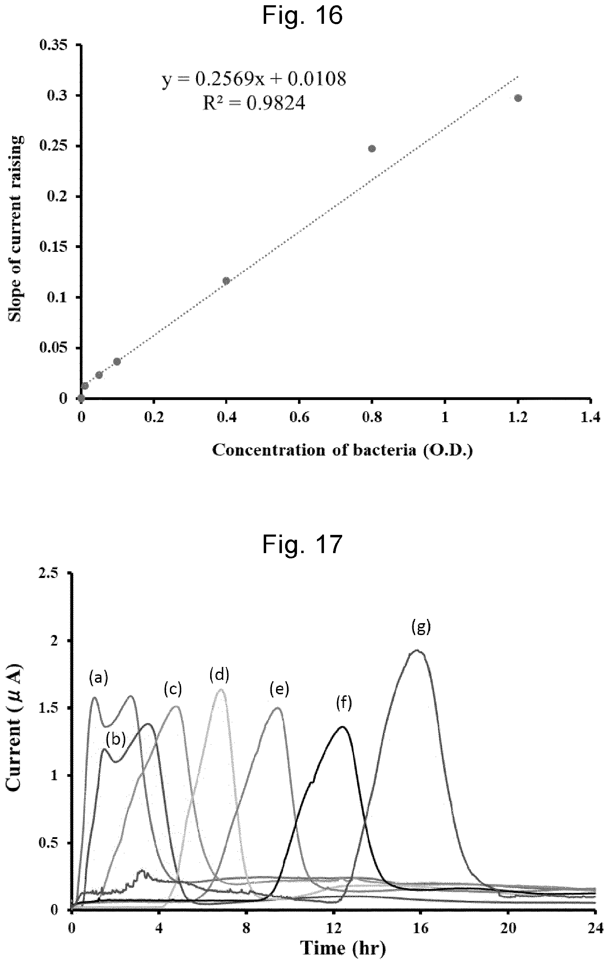

Fig. 16 is a graph showing a relationship between the concentration (OD₆₀₀ value) of MR-1 and the slope of a "time-current value" curve when the current value is raised.

From Fig. 16, the OD₆₀₀ value indicating the concentration of MR-1 and the slope of the curve described above were in a linear relationship.

### (Measurement Using Solid Electrolyte Containing Electron Transfer Substance)

Next, the same test was performed by adding riboflavin having a concentration of 0.004 g/mL to the LB agar medium.

Fig. 17 is a graph showing measurement results of current values (µA) for 24 hours from the start of the measurement by using the culture solutions having the OD₆₀₀ value of (a) 0.8 (6 × 10⁷ cells), (b) 0.4 (3 × 10⁷ cells), (c) 0.1 (7.5 × 10⁶ cells), (d) 0.01 (7.5 × 10⁵ cells), (e) 0.001 (7.5 × 10⁴ cells), (f) 0.0001 (7.5 × 10³ cells), and (g) 0.00001 (750 cells), as a culture solution smeared on a riboflavin-containing LB agar medium.

From Fig. 17, it was found that the time until the current value was raised from the start of the measurement was reduced as the concentration of MR-1 increased.

In addition, it was found that in a case where the concentrations of MR-1 were the same (OD₆₀₀ = 0.1), in the riboflavin-containing LB agar medium of the LB agar medium containing riboflavin and the LB agar medium not containing riboflavin, the time until the current value was raised from the start of the measurement was reduced.

These results indicate that the solid electrolyte contains a substance for accelerating the electron transfer function of the biological particles to be detected (the microorganisms), and thus, the responsiveness of the biosensor is improved, and the current value can be more rapidly measured by the measurement device according to the embodiment of the present invention.

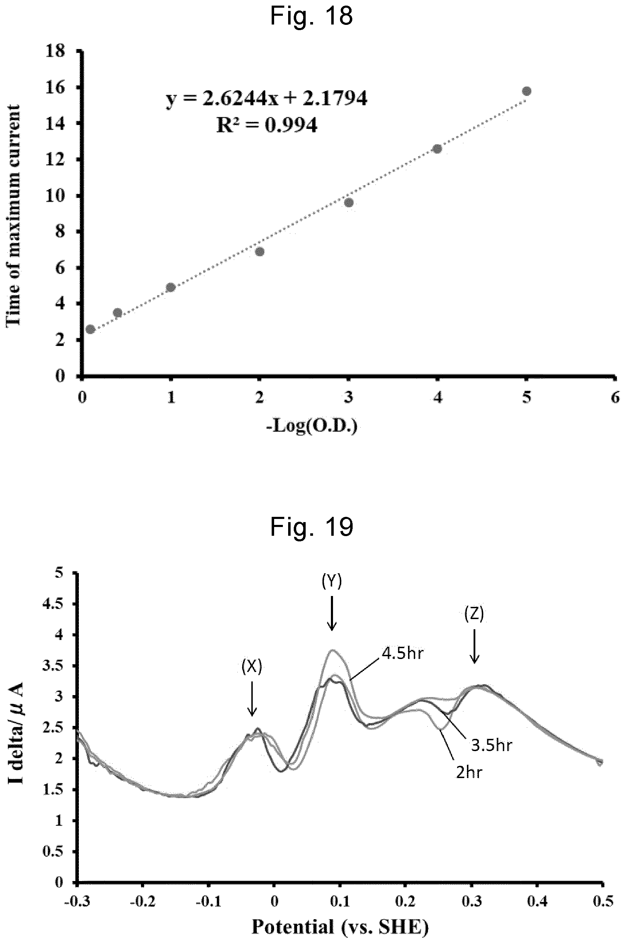

Fig. 18 is a graph showing a relationship between the concentration (a logarithm of the OD₆₀₀ value) of MR-1 and the time to reach a maximum value of the current value, of the results shown in Fig. 17 (in the case of using the riboflavin-containing LB agar medium).

From the fact that the graph shown in Fig. 18 was in a linear relationship, it was indicated that the current value measured by the measurement device according to the embodiment of the present invention was also in a correlation with an increase in the biological particles to be detected (proliferation behavior of the microorganisms).

### (Measurement Using Solid Electrolytes Containing Electron Transfer Substances Having Different Concentrations)

Next, the same test was performed by setting the concentration of riboflavin to be added to the LB agar medium to 0.014 g/mL, 0.004 g/mL, and 0.001 g/mL. A culture solution having an OD₆₀₀ value of 0.8 (6 × 10⁷ cells) was used as the MR-1 culture solution.

As a result thereof, a sharp rise of the current value was observed immediately after the start of the measurement, regardless of the concentration of riboflavin, and a change such as a difference in the maximum values of the current values due to an increase in the concentration of riboflavin was not particularly observed. In the concentration range set in this test example, a sufficient effect was checked in all cases.

### (Differential Pulse Voltammetry (DPV))

Fig. 19 is a graph showing a relationship between the potential (voltage value with respect to a standard hydrogen electrode (SHE)) at the time point of 2 hours, 3.5 hours, and 4.5 hours from the start of the measurement and the measured current (ΔI, unit: µA), in a case where the culture solution having the OD₆₀₀ value of 0.8 (6 × 10⁷ cells) is used as the MR-1 culture solution, shown in (a) of Fig. 17.

As shown in Fig. 19, curves obtained at three time points described above had high similarity, and a remarkable difference was not observed.

In Fig. 19, it is considered that peaks indicated by signs of (X), (Y), and (Z) respectively represent that electrons are transferred to the electrode of the biosensor from MR-1 trapped on the solid electrolyte, in the following three aspects:
(X) an aspect in which the electron transfer to the electrode from MR-1 is performed through riboflavin contained in the solid electrolyte;
(Y) an aspect in which the electron transfer to the electrode from the MR-1 is accelerated by adsorbing riboflavin contained in the solid electrolyte in membrane protein on the surface of MR-1; and
(Z) an aspect in which the electron transfer to the electrode from the MR-1 is performed without riboflavin contained in the solid electrolyte.

From the results of the test example as described above, in the present invention, microorganisms having an electron transfer function can be set as a target to be detected, and thus, the present invention can be expected to be applied to various applications including the plant and the medical instrument described above.

### Reference Signs List

- 100: Biosensor
- 101: First Substrate
- 101: Top Face of First Substrate
- 102: Solid Electrolyte
- 103: Substrate with Solid Electrolyte
- 104: Spacer
- 105: Second Substrate
- 106: First Electrode
- 107: Second Electrode
- 108: Substrate with Electrodes
- 213: Computing Part
- 400: Plant
- 401: Leaf
- 600: Body
- 601: Housing
- 602: Display
- 603: Operation Button
- 604: Sensor Insertion Slot
- 801: Connector
- 802: Plate
- 803: Extendable Rod
- 804: Cylinder
- 900: Measurement Device
- 901: Substrate
- 910: Output Part
- 911: Power Supply Device
- 912: Control Part
- 913: Computing Part
- 914: Detection Part
- 915: Display Unit
- 915: Display Part
- 916: Battery
- 917: Biosensor
- 918: Control Computer
- 919: Potentiostat (Voltage Applying Part)
- 1300: Substrate with Electrodes
- 1301: Third Electrode
- 1500, 1510: Tube
- 1501, 1511: Tube Body
- 1502: Joint Portion
- 1512: Ferrule Portion

## Claims

1. A measurement device, comprising
a biosensor (100, 917),
a voltage applying part (919) configured for applying a voltage between electrodes (106, 107) of the biosensor, the biosensor comprising a solid electrolyte (102) for trapping at least one type of biological particles having an electron transfer function selected from the group consisting of microorganisms existing on an object to be tested and particles derived from the microorganisms by being brought into contact with the object to be tested, and at least two electrodes, wherein a gap distance between the electrodes is less than or equal to the size of the biological particles and the electrodes are configured such that the solid electrolyte and the electrodes are in contact with each other,
a detection part (914) configured for measuring a current value flowing between the electrodes by the electron transfer function of the biological particles bridging the gap between the electrodes when the voltage is applied, and
a computing part (913) configured for providing information resulting from the microorganisms by comparing the current value with a reference value stored in advance in which a current value and at least one type of factor selected from the group consisting of the presence or absence of the biological particles, a proliferative state of the microorganisms, and the presence or absence of expression of pathogenicity of the microorganisms are defined;
**characterized in that** the solid electrolyte contains Riboflavin.

2. The measurement device according to claim 1, wherein the biosensor is a laminated body in which a substrate with solid electrolyte comprising a first substrate and the solid electrolyte disposed on the first substrate, and a substrate with electrodes comprising a second substrate and at least the two electrodes disposed on the second substrate are laminated such that the solid electrolyte and the electrodes are in contact with each other.

3. The measurement device according to claim 1 or 2, wherein the solid electrolyte is hydrogel.

4. The measurement device according to any one of claims 1 to 3, wherein the shortest distance between the electrodes is 50 nm to 10 µm.

5. The measurement device according to any one of claims 1 to 4, wherein at least one of the electrodes is a comb-shaped electrode having a combteeth shape.

6. The measurement device according to any one of claims 1 to 5, wherein the information resulting from the microorganisms is information for evaluating a risk of developing disease damage on a plant due to the microorganisms.

7. The measurement device according to any one of claims 1 to 6, wherein the biological particles are derived from at least one type of microorganisms selected from the group consisting of genus Phytophthora, genus Botrytis, genus Plasmopara, genus Sphaerotheca, genus Alternaria, genus Uromyces, genus Ustilago, genus Phakopsora, genus Burkholderia, genus Xanthomonadaceae, genus Xylella, genus Pseudomonas, genus Erwinia, genus Streptomyces, genus Candidatus Liberibacterasiaticus causing a disease damage on Citreae, and genus Candidatus Phlomobacter causing a disease damage on strawberries.

8. The measurement device according to claim 6 or 7, wherein the disease damage on the plant is at least one type selected from the group consisting of powdery mildew, a rust disease, a leaf spot, wilt, disease damage on roots and stems, disease damage on ears, rotten fruits, disease damage of seed and soilborne decay, crown wart, witch's broom, and a crinkle leaf.

9. The measurement device according to any one of claims 1 to 5, wherein the information resulting from the microorganisms is information for evaluating a cleanliness of a medical instrument.

10. The measurement device according to claim 9, wherein the medical instrument is an endoscope.

11. An evaluation method, comprising
a step of trapping at least one type of biological particles having an electron transfer function on a solid electrolyte (102) by bringing the solid electrolyte into contact with an object to be tested, the biological particles being selected from the group consisting of microorganisms existing on the object to be tested and particles derived from the microorganisms,
a step of forming a biosensor (100, 917) by preparing a substrate with electrodes comprising a substrate (105) and at least two electrodes (106, 107) disposed on the substrate, a gap distance between the electrodes being less than or equal to the size of the biological particles, and by disposing the solid electrolyte after contact on the substrate with electrodes to be in contact with both of the electrodes,
a step of measuring a current value to be obtained by the electron transfer function of the biological particles bridging the gap between the electrodes by applying a voltage between the electrodes, and
a step of obtaining information resulting from the microorganisms by comparing the current value with a reference value stored in advance in which a current value and at least one type of factor selected from the group consisting of the presence or absence of the biological particles, a proliferative state of the microorganisms, and the presence or absence of expression of pathogenicity of the microorganisms are defined;
**characterized in that** the solid electrolyte contains Riboflavin.

12. The evaluation method according to claim 11, wherein the information resulting from the microorganisms is information for evaluating a risk of developing disease damage on a plant due to the microorganisms.

13. The evaluation method according to claim 11, wherein the information resulting from the microorganisms is information for evaluating a cleanliness of a medical instrument.

14. The evaluation method according to claim 13, wherein the medical instrument is an endoscope.

## Patentansprüche

1. Messvorrichtung, umfassend
einen Biosensor (100, 917),
einen Spannungsanlegeteil (919), der zum Anlegen einer Spannung zwischen Elektroden (106, 107) des Biosensors konfiguriert ist, wobei der Biosensor einen Festelektrolyten (102) zum Einfangen mindestens einer Art von biologischen Partikeln, die eine Elektronentransferfunktion aufweisen, ausgewählt aus der Gruppe bestehend aus Mikroorganismen, die auf einem zu testenden Objekt vorhanden sind, und Partikeln, die von den Mikroorganismen stammen, indem sie mit dem zu testenden Objekt in Kontakt gebracht werden, und mindestens zwei Elektroden umfasst, wobei ein Spaltabstand zwischen den Elektroden kleiner oder gleich der Größe der biologischen Partikel ist und die Elektroden so konfiguriert sind, dass der Festelektrolyt und die Elektroden miteinander in Kontakt stehen,
einen Erfassungsteil (914), der zum Messen eines Stromwerts konfiguriert ist, der zwischen den Elektroden durch die Elektronentransferfunktion der biologischen Partikel fließt, die den Spalt zwischen den Elektroden überbrücken, wenn die Spannung angelegt wird, und
einen Rechenteil (913), der so konfiguriert ist, dass er Informationen bereitstellt, die sich aus den Mikroorganismen ergeben, indem er den aktuellen Wert mit einem im Voraus gespeicherten Referenzwert vergleicht, in dem ein aktueller Wert und mindestens eine Art von Faktor, ausgewählt aus der Gruppe bestehend aus dem Vorhandensein oder Fehlen der biologischen Partikel, einem Proliferationszustand der Mikroorganismen und dem Vorhandensein oder Fehlen einer Pathogenitätsausprägung der Mikroorganismen, definiert sind;
**dadurch gekennzeichnet, dass** der Festelektrolyt Riboflavin enthält.

2. Messvorrichtung nach Anspruch 1, wobei der Biosensor ein laminierter Körper ist, in dem ein Substrat mit Festelektrolyten, das ein erstes Substrat und den auf dem ersten Substrat angeordneten Festelektrolyten umfasst, und ein Substrat mit Elektroden, das ein zweites Substrat und mindestens die zwei auf dem zweiten Substrat angeordneten Elektroden umfasst, so laminiert sind, dass der Festelektrolyt und die Elektroden miteinander in Kontakt stehen.

3. Messvorrichtung nach Anspruch 1 oder 2, wobei der Festelektrolyt ein Hydrogel ist.

4. Messvorrichtung nach einem der Ansprüche 1 bis 3, wobei der kürzeste Abstand zwischen den Elektroden 50 nm bis 10 µm beträgt.

5. Messvorrichtung nach einem der Ansprüche 1 bis 4, wobei mindestens eine der Elektroden eine kammförmige Elektrode ist, die eine Kammzahnform aufweist.

6. Messvorrichtung nach einem der Ansprüche 1 bis 5, wobei die aus den Mikroorganismen resultierenden Informationen Informationen zum Bewerten eines Risikos der Entwicklung von Krankheitsschäden an einer Pflanze aufgrund der Mikroorganismen sind.

7. Messvorrichtung nach einem der Ansprüche 1 bis 6, wobei die biologischen Partikel von mindestens einer Art von Mikroorganismen stammen, die aus der Gruppe ausgewählt ist, bestehend aus Gattung Phytophthora, Gattung Botrytis, Gattung Plasmopara, Gattung Sphaerotheca, Gattung Alternaria, Gattung Uromyces, Gattung Ustilago, Gattung Phakopsora, Gattung Burkholderia, Gattung Xanthomonadaceae, Gattung Xylella, Gattung Pseudomonas, Gattung Erwinia, Gattung Streptomyces, Gattung Candidatus Liberibacterasiaticus, die einen Krankheitsschaden an Citreae verursacht, und Gattung Candidatus Phlomobacter, die einen Krankheitsschaden an Erdbeeren verursacht.

8. Messvorrichtung nach Anspruch 6 oder 7, wobei es sich bei dem Krankheitsschaden an der Pflanze um mindestens eine Art handelt, ausgewählt aus der Gruppe bestehend aus Mehltau, einer Rostkrankheit, einer Blattfleckenkrankheit, Welke, Krankheitsschaden an Wurzeln und Stängeln, Krankheitsschaden an Ähren, faulen Früchten, Krankheitschäden an Samen und bodenbürtiger Fäulnis, Kronenwarze, Hexenbesen und einem Kräuselblatt.

9. Messvorrichtung nach einem der Ansprüche 1 bis 5, wobei die aus den Mikroorganismen resultierenden Informationen Informationen zum Bewerten einer Sauberkeit eines medizinischen Instruments sind.

10. Messvorrichtung nach Anspruch 9, wobei das medizinische Instrument ein Endoskop ist.

11. Bewertungsverfahren, umfassend
einen Schritt zum Einfangen mindestens einer Art von biologischen Partikeln, die eine Elektronentransferfunktion aufweisen, auf einem Festelektrolyten (102), indem der Festelektrolyt mit einem zu testenden Objekt in Kontakt gebracht wird, wobei die biologischen Partikel aus der Gruppe ausgewählt werden, bestehend aus auf dem zu testenden Objekt vorhandenen Mikroorganismen und Partikeln, die von den Mikroorganismen stammen,
einen Schritt zum Bilden eines Biosensors (100, 917) durch Vorbereiten eines Substrats mit Elektroden, das ein Substrat (105) und mindestens zwei auf dem Substrat angeordnete Elektroden (106, 107) umfasst, wobei ein Spaltabstand zwischen den Elektroden kleiner oder gleich der Größe der biologischen Partikel ist, und durch Anordnen des Festelektrolyten nach Kontakt auf dem Substrat mit Elektroden, sodass er mit beiden Elektroden in Kontakt steht,
einen Schritt zum Messen eines Stromwerts, der durch die Elektronentransferfunktion der biologischen Partikel erhalten wird, die den Spalt zwischen den Elektroden überbrücken, indem eine Spannung zwischen den Elektroden angelegt wird, und
einen Schritt zum Erhalten von Informationen, die sich aus den Mikroorganismen ergeben, indem der aktuelle Wert mit einem im Voraus gespeicherten Referenzwert verglichen wird, in dem ein aktueller Wert und mindestens eine Art von Faktor, ausgewählt aus der Gruppe bestehend aus dem Vorhandensein oder Fehlen der biologischen Partikel, einem Proliferationszustand der Mikroorganismen und dem Vorhandensein oder Fehlen einer Pathogenitätsausprägung der Mikroorganismen, definiert sind;
**dadurch gekennzeichnet, dass** der Festelektrolyt Riboflavin enthält.

12. Bewertungsverfahren nach Anspruch 11, wobei die aus den Mikroorganismen resultierenden Informationen Informationen zum Bewerten des Risikos der Entwicklung von Krankheitsschäden an einer Pflanze aufgrund der Mikroorganismen sind.

13. Bewertungsverfahren nach Anspruch 11, wobei die aus den Mikroorganismen resultierenden Informationen Informationen zum Bewerten der Sauberkeit eines medizinischen Instruments sind.

14. Bewertungsverfahren nach Anspruch 13, wobei das medizinische Instrument ein Endoskop ist.

## Revendications

1. Dispositif de mesure, comprenant
un biocapteur (100, 917),
une partie d'application de tension (919) configurée pour appliquer une tension entre des électrodes (106, 107) du biocapteur, le biocapteur comprenant un électrolyte solide (102) pour piéger au moins un type de particules biologiques présentant une fonction de transfert d'électrons sélectionnée dans le groupe consistant en des micro-organismes existant sur un objet à tester et des particules dérivées des micro-organismes par mise en contact avec l'objet à tester, et au moins deux électrodes, dans lequel une distance d'espacement entre les électrodes est inférieure ou égale à la taille des particules biologiques et les électrodes sont configurées de telle sorte que l'électrolyte solide et les électrodes soient mutuellement en contact,
une partie de détection (914) configurée pour mesurer une valeur de courant circulant entre les électrodes par la fonction de transfert d'électrons des particules biologiques comblant l'espace entre les électrodes lorsque la tension est appliquée, et
une partie de calcul (913) configurée pour fournir des informations résultant des micro-organismes en comparant la valeur de courant avec une valeur de référence stockée à l'avance dans laquelle une valeur de courant et au moins un type de facteur sélectionné dans le groupe consistant en la présence ou l'absence des particules biologiques, un état prolifératif des micro-organismes et la présence ou l'absence d'expression du pouvoir pathogène des micro-organismes sont définis ;
**caractérisé en ce que** l'électrolyte solide contient de la riboflavine.

2. Dispositif de mesure selon la revendication 1, dans lequel le biocapteur est un corps stratifié dans lequel un substrat avec un électrolyte solide comprenant un premier substrat et l'électrolyte solide disposé sur le premier substrat, et un substrat avec des électrodes comprenant un second substrat et au moins les deux électrodes disposées sur le second substrat sont stratifiés de telle sorte que l'électrolyte solide et les électrodes soient mutuellement en contact.

3. Dispositif de mesure selon la revendication 1 ou 2, dans lequel l'électrolyte solide est un hydrogel.

4. Dispositif de mesure selon l'une quelconque des revendications 1 à 3, dans lequel la distance la plus courte entre les électrodes est de 50 nm à 10 µm.

5. Dispositif de mesure selon l'une quelconque des revendications 1 à 4, dans lequel au moins une des électrodes est une électrode en forme de peigne présentant une forme de dents de peigne.

6. Dispositif de mesure selon l'une quelconque des revendications 1 à 5, dans lequel les informations résultant des micro-organismes sont des informations permettant d'évaluer un risque de développer des dommages pathologiques sur une plante du fait des micro-organismes.

7. Dispositif de mesure selon l'une quelconque des revendications 1 à 6, dans lequel les particules biologiques proviennent d'au moins un type de micro-organismes sélectionné dans le groupe consistant en le genre Phytophthora, le genre Botrytis, le genre Plasmopara, le genre Sphaerotheca, le genre Alternaria, le genre Uromyces, le genre Ustilago, le genre Phakopsora, le genre Burkholderia, le genre Xanthomonadaceae, le genre Xylella, le genre Pseudomonas, le genre Erwinia, le genre Streptomyces, le genre Candidatus Liberibacterasiaticus provoquant des dommages pathologiques sur les Citreae et le genre Candidatus Phlomobacter provoquant des dommages pathologiques sur les fraises.

8. Dispositif de mesure selon la revendication 6 ou 7, dans lequel les dommages pathologiques sur la plante sont au moins un type sélectionné dans le groupe consistant en de l'oïdium, une maladie de la rouille, une tache foliaire, du flétrissement, des dommages pathologiques sur les racines et les tiges, des dommages pathologiques sur les épis, des fruits pourris, des dommages pathologiques par la pourriture des graines et du sol, la verrue couronnée, le balai de sorcière et une feuille froissée.

9. Dispositif de mesure selon l'une quelconque des revendications 1 à 5, dans lequel les informations résultant des micro-organismes sont des informations permettant d'évaluer la propreté d'un instrument médical.

10. Dispositif de mesure selon la revendication 9, dans lequel l'instrument médical est un endoscope.

11. Procédé d'évaluation, comprenant
une étape de piégeage d'au moins un type de particules biologiques présentant une fonction de transfert d'électrons sur un électrolyte solide (102) par mise en contact de l'électrolyte solide avec un objet à tester, les particules biologiques étant sélectionnées dans le groupe consistant en des micro-organismes existant sur l'objet à tester et des particules dérivées des micro-organismes,
une étape de formation d'un biocapteur (100, 917) par préparation d'un substrat avec des électrodes comprenant un substrat (105) et au moins deux électrodes (106, 107) disposées sur le substrat, une distance d'espacement entre les électrodes étant inférieure ou égale à la taille des particules biologiques, et en disposant l'électrolyte solide après contact sur le substrat avec des électrodes pour être en contact avec les deux électrodes,
une étape de mesure d'une valeur de courant devant être obtenue par la fonction de transfert d'électrons des particules biologiques comblant l'espace entre les électrodes en appliquant une tension entre les électrodes, et
une étape d'obtention d'informations résultant des micro-organismes par comparaison de la valeur de courant avec une valeur de référence stockée à l'avance dans lequel une valeur de courant et au moins un type de facteur sélectionné dans le groupe consistant en la présence ou l'absence des particules biologiques, un étant prolifératif des micro-organismes et la présence ou l'absence d'expression du pouvoir pathogène des micro-organismes sont définis ;
**caractérisé en ce que** l'électrolyte solide contient de la riboflavine.

12. Procédé d'évaluation selon la revendication 11, dans lequel les informations résultant des micro-organismes sont des informations permettant d'évaluer un risque de développer des dommages pathologiques sur une plante du fait des micro-organismes.

13. Procédé d'évaluation selon la revendication 11, dans lequel les informations résultant des micro-organismes sont des informations permettant d'évaluer la propreté d'un instrument médical.

14. Procédé d'évaluation selon la revendication 13, dans lequel l'instrument médical est un endoscope.
